(19) Europäisches Patentamt
European Patent Office
Office européen des brevets

(11) Veröffentlichungsnummer: **0 180 043**
**A2**

(12) # EUROPÄISCHE PATENTANMELDUNG

(21) Anmeldenummer: **85112310.9**

(22) Anmeldetag: **27.09.85**

(51) Int. Cl.[4]: **C 07 C 87/24**, C 07 C 87/29, C 07 C 87/451, C 01 B 25/455, A 61 K 7/22

(30) Priorität: **28.09.84 DE 3435675**

(43) Veröffentlichungstag der Anmeldung: **07.05.86 Patentblatt 86/19**

(84) Benannte Vertragsstaaten: **AT BE CH DE FR GB IT LI LU NL SE**

(71) Anmelder: **Benckiser-Knapsack GmbH, Dr. Albert-Reimann-Strasse 2, D-6802 Ladenburg (DE)**

(72) Erfinder: **Kleemann, Stephan, Dr. Dipl.-Chem., Mozartstrasse 25, D-6905 Schriesheim (DE)**
Erfinder: **Auel, Theodor, Dr. Dipl.-Chem., Winzerstrasse 3, D-6803 Edingen-Neckarhausen (DE)**
Erfinder: **Dany, Franz-Josef, Dr. Dipl.-Chem., Heddinghovener Strasse 47, D-5042 Erftstadt (DE)**

(74) Vertreter: **Patentanwälte Zellentin, Zweibrückenstrasse 15, D-8000 München 2 (DE)**

(54) **Ammoniummonofluorophosphate, Verfahren zu ihrer Herstellung und ihre Verwendung.**

(57) Die vorliegende Erfindung betrifft Ammoniummonofluorophosphate der allgemeinen Formel I

$$(R_1R_2R_3NH)R_4PO_3F \qquad (I)$$

in der $R_1$, $R_2$ und $R_3$ gleich oder verschieden sind und einen geradkettigen, verzweigten oder cyclischen, gesättigten oder ungesättigten Alkyl-, Aralkyl- oder Arylrest bedeuten, wobei diese gegebenenfalls noch durch Alkyl, Alkoxy oder Hydroxyl substituiert sein können und zwei der Substituenten auch Wasserstoff sein können oder zusammen eine Alkylenbrücke bilden,

$R_4$ ein Wasserstoffatom oder die Gruppe $R_5R_6R_7NH$ ist, in der $R_5$, $R_6$ und $R_7$ die gleichen Bedeutungen haben können wie $R_1$, $R_2$, $R_3$, sowie Verfahren zu deren Herstellung und deren Verwendung in Zahnpflegemitteln.

Die beanspruchten Substanzen zeichnen sich durch eine verbesserte Freisetzung von Fluor bei der Anwendung als Zahnpflegemittel aus.

EP 0 180 043 A2

PATENTANWÄLTE ZELLENTIN
D-8000 München 2, Zweibrückenstraße 15, Telefon 089-22 45 85, Telex 5-22 903, Fax 089-22 20 66

Benckiser-Knapsack GmbH
6802 Ladenburg

26. September 1985
Eu 85 417

# Ammoniummonofluorophosphate, Verfahren zu ihrer Herstellung und ihre Verwendung

Gegenstand der vorliegenden Erfindung sind Alkyl- und Arylammoniummonofluorophosphate, Verfahren zu ihrer Herstellung und ihre Verwendung als Zahnpastenadditive zur Kariesinhibierung.

Es ist bekannt, daß Fluoride, die in geringen Mengen Zahnpasten zugesetzt werden, imstande sind, die Zahnsubstanz zu härten und dadurch den Zahnverfall und Karies zu verringern. Da die meisten Fluoride, wie auch Flußsäure selbst, recht toxisch sind, ist es bekannt, stattdessen Natriummonofluorophosphat ($Na_2PO_3F$) zu verwenden, aus dem Fluorwasserstoff durch Hydrolyse freigesetzt wird.

Obwohl dieses Salz seit langem in großem Umfang als Zahnpastenadditiv verwendet wird, besteht doch das Bedürfnis nach anderen Additiven, die insbesondere während der Herstellung und Lagerung der Zahnpasten eine geringere Hydrolyse aufweisen und erst bei der Anwendung als Zahnpasta in gewünschtem Maße Fluorwasserstoff freisetzen.

Es stellte sich daher die Aufgabe, neue oder/und als Zahnpastaadditive bessere Fluorwasserstoff abgebende Verbindungen zu finden.

Überraschenderweise wurde nun gefunden daß Ammoniummonofluorophosphate der allgemeinen Formel I

$$(R_1R_2R_3NH)R_4PO_3F \qquad (I)$$

in der $R_1$, $R_2$ und $R_3$ gleich oder verschieden sind und einen geradkettigen, verzweigten oder cyclischen, gesättigten oder ungesättigten Alkyl-, Aralkyl- oder Arylrest bedeuten, wobei diese gegebenenfalls noch durch Alkyl, Alkoxy oder Hydroxyl substituiert sein können und zwei der Substituenten auch Wasserstoff sein können oder zusammen eine Alkylenbrücke bilden,

$R_4$ ein Wasserstoffatom oder die Gruppe $R_5R_6R_7NH$ ist

in der $R_5$, $R_6$ und $R_7$ die gleichen Bedeutungen haben können wie $R_1$, $R_2$, $R_3$.

in diesem Sinne verwendet werden können. Bevorzugt sind sekundäre und tertiäre ungesättigte Amine enthaltende symmetrische Ammoniummonofluorophosphate sowie Ammoniummonofluorohydrogenphosphate der allgemeinen Formel I

$$(R_1R_2R_3NH)R_4\ PO_3F \qquad (I)$$

in der $R_1$, $R_2$ und $R_3$ gleich oder verschieden sind und einen geradkettigen, verzweigten oder cyclischen, ungesättigten Alkyl-, Aralkyl- oder Arylrest bedeuten, wobei diese gegebenenfalls noch durch Alkyl, Alkoxy oder Hydroxyl substituiert sein können oder zusammen eine Alkylenbrücke bilden, $R_3$ auch ein Wasserstoffatom sein kann und $R_4$ ein Wasserstoffatom oder die Gruppe $R_5R_6R_7NH$ ist, in der $R_5$, $R_6$ und $R_7$ die gleichen Bedeutungen haben wie $R_1$, $R_2$, $R_3$, die bisher nicht beschrieben sind sowie die neuen unsymmetrischen Ammoniummonofluorophosphate der allgemeinen Formel I, in der $R_1$, $R_2$ und $R_3$ gleich oder verschieden sind und einen geradkettigen, verzweigten oder cyclischen, gesättigten oder ungesättigten Alkyl-, Aralkyl- oder Arylrest bedeuten, wobei diese gegebenenfalls noch durch

Alkyl, Alkoxy oder Hydroxyl substituiert sein können und zwei der Substituenten auch Wasserstoff sein können oder zusammen eine Alkylenbrücke bilden, $R_4$ die Gruppe $R_5R_6R_7NH$ ist, in der $R_5$, $R_6$ und $R_7$ nicht die gleichen Bedeutungen haben können wie $R_1$, $R_2$, $R_3$,
Für die erfindungsgemäße Verwendung als Zahnpastaadditiv ist es wichtig, daß die hydrolytische Spaltung der Phosphor-Fluor-Bindung bei diesen Verbindungen im Mundbereich langsam aber kontinuierlich abläuft, so daß eine niedere, jedoch konstante Konzentration an freien Fluoriden erhalten wird. Es wird angenommen, daß die gegenüber Natriummonofluorophosphat größere hydrolytische Stabilität der Phosphor-Fluor-Bindung auf die hydrophoben Substituenten der Ammoniumionen zurückzuführen ist.

Während sekundäre und tertiäre ungesättigte Amine enthaltende symmetrische Ammoniummonofluorophosphate sowie Ammoniummonfluorohydrogenphosphate den großen Vorteil bieten, mehrere verschiedene Reste R am gleichen Stickstoffatom zu vereinigen, wird diese Möglichkeit bei den unsymmetrischen Ammoniummonofluorophosphaten zusätzlich durch die Möglichkeit der Kombination verschiedener Amine erweitert.
Dadurch ergibt sich ein breites Spektrum an Anwendungsmöglichkeiten, wie beispielsweise die Kombination von Resten bzw. Aminen mit guter Hafteigenschaft auf der Mundschleimhaut, mit Resten variabler Hydrophobie und damit unterschiedlich schneller Fluorid-Freisetzung.

Mit Hilfe der erfindungsgemäßen neuen Verbindungen können deshalb die verschiedenen Ansprüche an Zahnpasten mit beispielsweise großer Langzeitwirkung (für abends), sowie mit mäßig schneller Fluoridfreisetzung bei gleichzeitig guter Haftung (für morgens und mittags), in einfacher Art und Weise realisiert werden

Aus der USP 3,507,918 sind bereits gesättigte Amine enthaltende symmetrische Di(alkylammonium)monofluorophosphate sowie gesättigte Amine enthaltende Mono(alkylammonium)-monofluorohydrogenphosphate bekannt, die als Öl-lösliche Schmiermitteladditive verwendet werden. Niedermolekulare Verbindungen dieser Art werden weiterhin als Stickstoff- und Phosphorlieferanten zur Bodenkonservierung und Düngung verwendet. Zur Herstellung dieser Verbindungen soll gemäß Kirk-Othmer, 3. Auflage, Vol. 10, S. 782 aus Orthophosphorsäure und Flußsäure hergestellte Monofluorophosphorsäure in eine äquivalente Menge eines entsprechenden Amins, welches in einem leicht siedenden inerten Lösemittel gelöst ist, eingetragen werden. Da die so hergestellte Monofluorophosphorsäure in Gegenwart von Wasser mit den Ausgangskomponenten Orthophosphorsäure und Flußsäure im Gleichgewicht steht (Kirk-Othmer, 3. Auflage, Vol. 10, S. 780-81), erhält man auf diese Weise nach Abdampfen des Lösemittels ein Salzgemisch aus dem gewünschten Ammoniummonofluorophosphat, Ammoniumphosphat und Ammoniumfluorid, welches insbesondere wegen des Gehaltes an freiem Fluorid als Zahnpastenadditiv wenig geeignet ist.

Darüber hinaus bereitet die Handhabung von Monofluorophosphorsäure sowie freier Flußsäure Schwierigkeiten bei der Herstellung, so daß es eine weitere Aufgabe der Erfindung war, neue Verfahren zu finden, nach denen sich allgemeine Verbindungen der Formel I in der $R_1$, $R_2$ und $R_3$ gleich oder verschieden sind und einen geradkettigen, verzweigten oder cyclischen, gesättigten oder ungesättigten Alkyl-, Aralkyl- oder Arylrest bedeuten, wobei diese gegebenenfalls noch durch Alkyl, Alkoxy oder Hydroxyl substituiert sein können und zwei der Substituenten auch Wasserstoff sein können oder zusammen eine Alkylenbrücke bilden, $R_4$ ein Wasserstoffatom oder die Gruppe $R_5R_6R_7NH$ ist, in der

$R_5$, $R_6$ und $R_7$ die gleichen Bedeutungen haben können wie $R_1$, $R_2$, $R_3$, auf einfachere Weise und frei von Verunreinigungen durch andere Salze herstellen lassen.

Überraschenderweise läßt sich dieses in einfacher Weise durch Umsetzung der gut charakterisierten anorganischen Salze der Monofluorophosphorsäure, vorzugsweise des Dinatriummonofluorophosphates mit den entsprechenden Aminen bzw. ihren Salzen, vorzugsweise den Hydrochloriden, durchführen.

Bevorzugt wird dabei das Natriummonofluorophosphat mit dem entsprechenden Aminhydrochlorid umgesetzt, wobei überraschenderweise eine nahezu quantitative Umsalzung unter Bildung der Ammoniummonofluorophosphate stattfindet. Die Reaktion wird durch Zusammenmischen der Komponenten in einem Lösemittel bei Raumtemperatur oder bis zum Siedepunkt des Lösemittels erhöhter Temperatur durchgeführt. Als Lösemittel kommen sowohl Wasser als auch polare organische Lösemittel wie Aceton, Methylisobutylketon, Methanol, Ethanol, Propanol, Butanol etc., aber auch Gemische dieser Lösemittel, gegebenenfalls unter Zusatz von unpolaren organischen Lösemitteln wie Toluol, in Frage.

Insbesondere zur Herstellung der höher molekularen Verbindungen wird Wasser oder ein wäßriger Alkohol bevorzugt, da die gewünschten Ammoniummonofluorophosphate sich daraus schwerlöslich abscheiden, während die Ausgangskomponenten und das ebenfalls gebildete Natriumchlorid in Lösung verbleiben und somit leicht abgetrennt werden können. Niedermolekulare Ammoniummonofluorophosphate, die in Wasser bzw. wäßrigem Alkohol in stärkerem Maße löslich sind, lassen

sich auf diese Weise gewinnen, indem man nach erfolgter Umsetzung das Lösemittel abzieht und aus dem Rückstand das Ammoniummonofluorophosphat mit einem wasserfreien organischen Lösemittel extrahiert, wobei das Natriumchlorid zurückbleibt, und durch Abziehen des organischen Lösemittels das reine Ammoniummonofluorophosphat gewinnt.

Eine zusätzliche Reinigung des Produktes kann durch Umkristallisation, beispielsweise aus einem niederen Alkohol, erfolgen. Alternativ läßt sich die Umsetzung auch von vornherein in einem solchen organische Lösemittel durchführen, wobei das entstehende Natriumchlorid als schwer lösliches Reaktionsprodukt ausfällt und zusammen mit nicht umgesetzten Ausgangsprodukten leicht abgetrennt werden kann. Das Ammoniummonofluorophosphat wird dann durch Abdampfen des Lösemittels gewonnen. Nachteilig bei diesem Verfahren ist es, daß die Ausgangskomponenten in diesen Lösemitteln ebenfalls nur wenig löslich sind, so daß die Umsetzung längere Zeit in Anspruch nimmt und bei vorzugsweise höheren Temperaturen durchgeführt werden muß.

Bevorzugt wird die Umsetzung in organischen Lösemitteln jedoch zur Herstellung der bisher nicht beschriebenen unsymmetrischen Ammoniummonofluorophosphate der allgemeinen Formel I verwendet, in der $R_1$, $R_2$ und $R_3$ gleich oder verschieden sind und einen geradkettigen, verzweigten oder cyclischen, gesättigten oder ungesättigten Alkyl-, Aralkyl- oder Arylrest bedeuten, wobei diese gegebenenfalls noch durch Alkyl, Alkoxy oder Hydroxyl substituiert sein können und zwei der Substituenten auch Wasserstoff sein können oder zusammen eine Alkylenbrücke bilden, $R_4$ die Gruppe $R_5R_6R_7NH$ ist, in der $R_5$, $R_6$ und $R_7$nicht die gleichen Bedeutungen haben können wie $R_1$, $R_2$, $R_3$ , da,

insbesondere wenn beide Ammoniumgruppen sich in ihrer Größe und/oder Hydrophilie stark unterscheiden, bei der direkten Ausfällung aus wäßriger oder wäßrig alkoholischer Lösung zunächst nur das schwerer lösliche der beiden möglichen Ammoniummonofluorophosphate ausfällt und erst wenn das entsprechende Amin verbraucht ist, auch das andere Amin ebenfalls unter Bildung eines Salzes reagiert.

Bei Verwendung eines organischen Lösemittels, in dem die entsprechenden Ammoniummonofluorophosphate löslich sind, gelingt es im Gegensatz dazu, die Salze in Lösung herzustellen und durch Abdampfen des Lösemittels zu erhalten.

Weiterhin wurde gefunden, daß man durch Umsetzen von Dinatriummonofluorophosphat mit sauren, vorzugsweise stark sauren Ionenaustauschern in wäßriger Lösung Monofluorophosphorsäure in hoher Reinheit erhalten kann, die bei sofortiger Umsetzung mit den entsprechenden Aminen reine Ammoniummonofluorophosphate ergibt. Da bei der Reaktion eine erhebliche Neutralisationswärme frei wird, muß die Lösung gekühlt werden, wobei Reaktionstemperaturen von 0 bis 60°C, vorzugsweise 10 bis 30°C und insbesondere 15 bis 20°C, bevorzugt werden. Das Amin bzw. Amingemisch wird dabei als Lösung oder Suspension in Wasser oder einem mit Wasser mischbaren organischen Lösemittel, wie beispielsweise Aceton, Methylisobutylketon, einem niederen Alkohol oder einer Mischung derselben, vorgelegt. Zur Vermeidung lokaler Überhitzung muß die Zugabe des Ionenaustauschereluates unter kräftigem Rühren erfolgen. Nach erfolgter Zugabe des stöchiometrische Mengen Monofluorophosphorsäure enthaltenden Ionenaustauschereluates, wird die Mischung zur vollständigen Umsetzung noch eine entsprechende Zeit weitergerührt. Die Reaktionsdauer liegt zwischen 15 Minuten bis 6 Stunden, vorzugsweise zwischen 1 und 3 Stunden. Soweit nicht in Wasser als Lösemittel gearbeitet wurde, kann durch Zugabe eines entsprechenden Überschusses von Wasser das Reaktionsprodukt zur vollständigen Ausfällung gebracht werden. Die wasserlöslichen niedermolekularen Ammoniummonofluorophosphate werden durch Abziehen des Lösemittels im Vakuum und Umkristallisation des Rückstandes in einem geeigneten Lösemittel, wie beispielsweise in einem niederen Alkohol oder Toluol, gewonnen. Die Produkte können anschließend durch Trocknen im Vakuum von anhaftenden letzten Spuren der Lösemittel befreit werden.

Die erfindungsgemäßen Alkyl- und Arylammoniummonofluorophosphate der allgemeinen Formel I sind feste bis wachsartige Substanzen, die je nach Art der Substituenten eine merkliche bis sehr geringe Löslichkeit in Wasser besitzen. Sie sind jedoch, insbesondere unter Zusatz oberflächenaktiver Stoffe, in Wasser dispergierbar. Der pH-Wert einer 1%-igen wäßrigen Lösung oder Dispersion dieser Verbindungen liegt bei pH 5-7. In Alkohol sind die erfindungsgemäßen Monofluorophosphate in der Wärme gut löslich.

Als Zahnpastenadditive werden die erfindungsgemäßen Verbindungen in einer Menge von 100 bis 5000 ppm, vorzugsweise 500 bis 2000 ppm Fluoridäquivalenten, d.h. etwa bis 10 Gew.% zugesetzt.

Unter symmetrischen Ammoniummonofluorophosphaten im Sinne der Erfindung werden Verbindungen der allgemeinen Formel I verstanden, bei denen sich $R_1R_2R_3NH$ und $R_4$ in ihrer Bedeutung entsprechen. Bei unsymmetrischen Verbindungen im Sinne der Erfindung können $R_1R_2R_3NH$ und $R_4$ nicht die gleiche Bedeutung haben.

Unter Alkylgruppen im Sinne der Erfindung werden Gruppen mit bis zu 30 C-Atomen, insbesondere 10-22 C-Atomen verstanden. Diese Gruppen können gegebenenfalls 1 oder 2 Doppelbindungen enthalten.

Cycloalkylgruppen können Monocyclen mit 5-8 C-Atomen, insbesondere c-Pentyl und c-Hexyl sein, aber auch Bicyclen, wie Tetrahydronaphtalin und Decalin.

Unter Aryl auch in Aralkyl sind Reste mit 6-14 C-Atomen, insbesondere Phenyl und Naphtyl, zu verstehen.

Soweit Alkyl- oder Alkoxygruppen als Substituenten der vorstehenden Reste auftreten, sind Gruppen mit 1-6 C-Atomen, insbesondere Methyl und Ethyl bzw. Methoxy und Ethoxy bevorzugt.

Die erfindungsgemäß hergestellten Substanzen können in symmetrischer oder unsymmetrischer Form, oder aber auch als Mischungen von symmetrischen mit unsymmetrischen Ammoniummonofluorophosphaten bzw. Ammoniummonofluorohydrogenphosphaten vorliegen.

## Beispiel 1

53,5 g Oleylamin werden innerhalb von 15 Minuten unter starkem Rühren und Kühlen auf 30°C mit 19,7 g 37 %iger HCl versetzt. Es werden weitere 500 ml $H_2O$ zugesetzt und dann unter fortdauerndem Rühren 14,4 g $Na_2PO_3F$, gelöst in 20 ml $H_2O$, zugesetzt.

Die Lösung wird weitere 2 Stunden bei 40°C gerührt, danach das schaumartige weiße Produkt mittels Filtration abgetrennt und kurz mit $H_2O$ gewaschen.

Die anschließende Trocknung im Vakuum bei 40°C ergibt 50,5 g Bis(oleylammonium)monofluorophosphat, d.s. 79,6 % der Theorie bezogen auf eingesetztes Oleylamin.

## Beispiel 2

72,0 g $Na_2$ $PO_3$ F werden in 1 Liter $H_2O$ gelöst und anschließend über einen regulierten und neutral gewaschenen Kationenaustauscher ( z.B. Lewatit® S 100 ) gegeben. Das Eluat wird unter starkem Rühren und Kühlen auf 20 bis 30°C in eine Emulsion von 267,5 g Oleylamin in 500 ml Aceton getropft. Die erhaltene weiße Suspension wird 30 Minuten nachgerührt und dann das Produkt mittels Filtration vom Lösemittel getrennt. Der wachsartige Rückstand wird 2x mit je 2000 ml Aceton gewaschen und bei 40°C im Vakuum getrocknet.

Die Ausbeute an analysenreinem Bis(oleylammonium)monofluorophosphat beträgt 297,2 g, d.s. 93,6 % der Theorie, bezogen auf eingesetztes Oleylamin.

## B e i s p i e l   3

65,7 g Decylamin werden in 200 ml $H_2O$ suspensiert und anschließend innerhalb von 15 Minuten unter starkem Rühren und Kühlen auf 30°C mit 41,1 g 37 %iger HCl versetzt. Anschließend werden unter fortdauerndem Rühren 30,0 g $Na_2PO_3F$, gelöst in 100 ml $H_2O$, zugesetzt.

Die Lösung wird weitere 3 Stunden bei 40°C gerührt, danach das schaumartige weiße Produkt mittels Filtration abgetrennt, kurz mit $H_2O$ und dann 2x mit je 300 ml Aceton gewaschen.

Die anschließende Trocknung im Vakuum bei 40°C ergibt 80,2 g Bis(decylammonium)monofluorophosphat, d.s. 93,1 % der Theorie bezogen auf eingesetztes Decylamin.

## B e i s p i e l   4

112,3 g Stearylamin werden in 2000 ml $H_2O$ aufgeschlämmt und dann innerhalb von 30 Minuten unter starkem Rühren und Kühlen auf 30°C mit 208,4 ml 2N HCl versetzt. Anschließend werden unter fortdauerndem Rühren 30,0 g $Na_2PO_3F$, gelöst in 100 ml $H_2O$, zugesetzt.

Die Lösung wird weitere 2 Stunden bei 40°C gerührt, danach das schaumartige weiße Produkt mittels Filtration abgetrennt, kurz mit $H_2O$ und dann mit insgesmt 1500 ml Aceton gewaschen.

Die anschließende Trocknung im Vakuum bei 40°C ergibt 132,9 g Bis(stearylammonium)monofluorophosphat, d.s. 99,8 % der Theorie bezogen auf eingesetztes Stearylamin.

Beispiel 5

65,8 g Docosylamin werden auf ca. 100°C erwärmt, heiß in 1000 ml $H_2O$ suspendiert und dann innerhalb von 30 Minuten unter starkem Rühren und Kühlen auf 30°C mit 104,2 ml 2N HCl versetzt. Anschließend werden unter fortdauerndem Rühren 15,0 g $Na_2PO_3F$, gelöst in 50 ml $H_2O$, zugesetzt.

Die Lösung wird weitere 2 Stunden bei 40°C gerührt, danach das schaumartige weiße Produkt mittels Filtration abgetrennt und kurz mit $H_2O$ und 1000 ml Methylisobutylketon gewaschen.

Die anschließende Trocknung im Vakuum bei 40°C ergibt 76,5 g Bis(docosylammonium)monofluorophosphat, d.s. 97,7 % der Theorie bezogen auf eingesetztes Docosylamin.

Beispiel 6

112,4 g Stearylamin und 111,5 g Oleylamin werden in 2,5 l $H_2O$ suspendiert und dann innerhalb von 15 Minuten unter starkem Rühren und Kühlen auf 30°C mit 416,9 ml 2N HCl versetzt. Anschließend werden unter fortdauerndem Rühren 60,0 g $Na_2PO_3F$, gelöst in 200 ml $H_2O$, zugesetzt.

Die Lösung wird weitere 2 Stunden bei 40°C gerührt, danach das schaumartige weiße Produkt mittels Filtration abgetrennt und kurz mit $H_2O$ sowie insgesamt 3000 ml Aceton gewaschen.

Die anschließende Trocknung im Vakuum bei 40°C ergibt 230,3 g Oleylammonium-stearylammoniummonofluorophosphat, d.s. 86,7 % der Theorie bezogen auf eingesetztes Amin.

## Beispiel 7

57,1 g Octadecylamin und 51,3 g Cetylamin werden innerhalb von 15 Minuten unter starkem Rühren und Kühlen auf 30° C mit 41,1 g 37 %iger HCl versetzt. Es werden weitere 1000 ml $H_2O$ zugesetzt und dann unter fortdauerndem Rühren 30,0 g $Na_2PO_3F$, gelöst in 100 ml $H_2O$, zugesetzt.

Die Lösung wird weitere 2 Stunden bei 40°C gerührt, danach das schaumartige weiße Produkt mittels Filtration abgetrennt und kurz mit $H_2O$ sowie insgesamt 1500 ml Aceton gewaschen.

Die anschließende Trocknung im Vakuum bei 40°C ergibt 93,9 g Cetylammonium-octadecylammoniummonofluorophosphat, d.s. 76,9 % der Theorie bezogen auf eingesetztes Amin.

## Beispiel 8

58,6 g Oleylamin und 52,2 g Dioctylamin werden innerhalb von 15 Minuten unter starkem Rühren und Kühlen auf 30° C mit 208,4 ml 2N HCl versetzt. Anschließend werden unter fortdauerndem Rühren 30,0 g $Na_2PO_3F$, gelöst in 100 ml $H_2O$, zugesetzt.

Die Lösung wird weitere 2 Stunden bei 40°C gerührt, danach das schaumartige weiße Produkt mittels Filtration abgetrennt und kurz mit $H_2O$ gewaschen.

Die anschließende Trocknung im Vakuum bei 40°C ergibt 98,5 g Oleylammonium-dioctadecylammoniummonofluorophosphat, d.s. 81,4 % der Theorie bezogen auf eingesetztes Amin.

Beispiel 9

72,0 g $Na_2PO_3F$ werden in 1 Liter $H_2O$ gelöst und anschließend über einen regulierten und neutral gewaschenen Kationenaustauscher (z.B. Lewatit® S100) gegeben. Das Eluat wird unter starken Rühren und Kühlen auf 20 bis 30°C in eine Mischung von 155,2 g Triethanolamin in 500 ml Aceton getropft. Die erhaltene Lösung wird 30 Minuten nachgerührt und dann der Ionenaustauscher mit insgesamt 2,5 Liter $H_2O$ gespült. Die resultierende wässrige Lösung wird im Vakuum bei 50°C bis zur Trockne eingeengt, der Rückstand in 250 ml Methanol gelöst und anschließend in 3 Liter Isopropanol eingerührt. Nach 48 Stunden wird das wachsartige Produkt mittels Filtration abgetrennt und über $P_2O_5$ getrocknet. Nach einer weiteren Fällung des restlichen Produkts aus dem Isopropanol-Filtrat werden insgesamt 182,1 g, d.s. 87,9 % der Theorie an Bis(triethanolammonium)monofluorophosphat erhalten.

PATENTANWÄLTE
ZELLENTIN
ZWEIBRÜCKENSTR. 15
8000 MÜNCHEN 2



Benckiser-Knapsack GmbH
6802 Ladenburg

26. September 1985
Eu 85 417

Patentansprüche

1. Sekundäre und tertiäre ungesättigte Amine enthaltende symmetrische Ammoniummonofluorophosphate sowie sekundäre oder tertiäre ungesättigte Amine enthaltende Ammoniummonofluorohydrogenphosphate der allgemeinen Formel I

$$(R_1R_2R_3NH)R_4\ PO_3F \qquad (I)$$

in der $R_1$, $R_2$ und $R_3$ gleich oder verschieden sind und einen geradkettigen, verzweigten oder cyclischen, ungesättigten Alkyl-, Aralkyl- oder Arylrest bedeuten, wobei diese gegebenenfalls noch durch Alkyl, Alkoxy oder Hydroxyl substituiert sein können oder zusammen eine Alkylenbrücke bilden, $R_3$ auch ein Wasserstoffatom sein kann und $R_4$ ein Wasserstoffatom oder die Gruppe $R_5R_6R_7NH$ ist, in der $R_5$, $R_6$ und $R_7$ die gleichen Bedeutungen haben wie $R_1$, $R_2$, $R_3$, sowie

unsymmetrische Ammoniummonofluorophosphate der allgemeinen Formel I, in der $R_1$, $R_2$ und $R_3$ gleich oder verschieden sind und einen geradkettigen, verzweigten oder cyclischen, gesättigten oder ungesättigten Alkyl-, Aralkyl- oder Arylrest bedeuten, wobei diese gegebenenfalls noch durch Alkyl, Alkoxy oder Hydroxyl substituiert sein können und zwei der Substituenten auch Wasserstoff sein können oder zusammen eine Alkylenbrücke bilden, und $R_4$ die Gruppe $R_5R_6R_7NH$ ist, in der $R_5$, $R_6$ und $R_7$ nicht alle die gleiche Bedeutung haben können wie $R_1$, $R_2$ und $R_3$.

2. Verfahren zur Herstellung von Ammoniummonofluorophosphaten sowie Ammoniummonofluorohydrogenphosphaten der allgemeinen Formel I, in der $R_1$, $R_2$ und $R_3$ gleich oder verschieden sind und einen geradkettigen, verzweigten oder cyclischen, gesättigten oder ungesättigten Alkyl-, Aralkyl- oder Arylrest bedeuten, wobei diese gegebenenfalls noch durch Alkyl, Alkoxy oder Hydroxyl substituiert sein können und zwei der Substituenten auch Wasserstoff sein können oder zusammen eine Alkylenbrücke bilden, $R_4$ ein Wasserstoffatom oder die Gruppe $R_5R_6R_7NH$ ist, in der $R_5$, $R_6$ und $R_7$ die gleichen Bedeutungen haben können wie $R_1$, $R_2$, $R_3$, dadurch gekennzeichnet, daß man

a) ein Salz der Monofluorophosphorsäure in einem Lösemittel mit einem oder mehreren Ammoniumsalzen der allgemeinen Formel II,

$$R_1R_2R_3NHX \qquad (II)$$

in der $R_1$, $R_2$ und $R_3$ die oben genannten Bedeutungen Bedeutungen haben und X das Anion einer Mineralsäure darstellt, umsetzt oder

b) ein Salz der Monofluorophosphorsäure mit einem sauren Ionenaustauscher in wäßriger Lösung in die freie Monofluorophosphorsäure überführt und diese sofort mit einer Lösung des oder der entsprechenden Amine der allgemeinen Formel II neutralisiert und die entstehende Verbindung der allgemeinen Formel I isoliert.

3. Verfahren gemäß Anspruch 2, dadurch gekennzeichnet, daß Dinatriummonofluorophosphat verwendet wird.

4. Verfahren gemäß Anspruch 2a, dadurch gekennzeichnet, daß X ein Chloridion darstellt.

5. Verfahren gemäß Anspruch 3 - 4, dadurch gekennzeichnet, daß in Wasser oder wäßrigen Alkoholen als Lösemittel gearbeitet wird.

6. Verfahren gemäß einem der Anspruch 2 - 5, dadurch gekennzeichnet, daß die Umsetzung bei Temperaturen zwischen 0° und dem Siedepunkt des verwendeten Lösemittels durchgeführt wird.

7. Verfahren gemäß Anspruch 6, dadurch gekennzeichnet, daß bei Raumtemperatur gearbeitet wird.

8. Verwendung von Ammoniummonofluorophosphaten gemäß Anspruch 1 u. 2, wobei diese auch in Form von Mischungen, und insbesondere in Form von Mischungen von symmetrischen Ammoniummonofluorophosphaten sowie Ammoniummonofluorohydrogenphosphaten mit unsymmetrischen Ammoniummonofluorophosphaten vorliegen können, als Zahnpastenadditiv.

9. Zahnpasta, enthaltend Ammoniummonofluorophosphat gemäß Anspruch 1, oder Mischungen von Ammoniummonofluorophosphaten gemäß Anspruch 8.